# EUROPEAN PATENT APPLICATION

(11) **EP 4 793 621 A1**
(43) Date of publication of application: **19.08.2026**
(21) Application number: 24874806.3
(22) Date of filing: 15.10.2024
(51) Int. Cl.: G01N 3/40, G01N 3/42, G01N 11/00, G01N 33/02

(54) **MEASUREMENT DEVICE, MEASUREMENT METHOD, AND PROGRAM**

(30) Priority: 02.10.2023 JP 2023171206
(71) Applicant: Fingervision Inc., Tokyo 135-0016 (JP)
(72) Inventor: YAMAGUCHI Akihiko, Tokyo 135-0016 (JP)
(74) Representative: Plougmann Vingtoft a/s
(86) International application number: PCT/JP2024/036629
(87) International publication number: WO 2025/075207

(57) **Abstract**

The present invention comprises: a data acquisition unit 241 that acquires position time series data indicating positions of a contact part 11 of a measurement unit 1 that comes into contact with an object W to be measured, said positions occurring during a measurement period when the contact part 11 is pressed against the object W to be measured, and pressure time series data indicating pressure applied to the contact part 11 during the measurement period; and a state identification unit 242 that analyzes the pressure time series data and the position time series data to identify an internal state parameter of the object W to be measured.

## Description

### TECHNICAL FIELD

The present invention relates to a measurement apparatus, a measurement method, and a program for measuring an internal state of a measurement target.

### BACKGROUND OF THE INVENTION

Conventionally, a method is known in which the hardness of a measurement target is measured by measuring a reaction force generated by pressing an indenter into the measurement target. In conventional measurement methods, the hardness of the measurement target is evaluated by comparing the measured reaction force per contact area with a reference value (for example, see Patent Document 1).

### PRIOR ART

### PATENT DOCUMENT

[Patent Document 1] Japanese Unexamined Patent Application Publication No. 2006-250633

### SUMMARY OF INVENTION

### PROBLEMS TO BE SOLVED BY THE INVENTION

There are cases where it is necessary to grasp the internal state of an object whose interior is not homogeneous. Although the hardness of the surface of the measurement target can be measured using conventional measurement methods, when the interior of the measurement target is not homogeneous, as in the case of food, it has not been possible to measure the distribution of the internal state of the measurement target.

The present invention has been made in view of these points, and its object is to make it possible to identify the distribution of the internal state of a measurement target.

### MEANS FOR SOLVING THE PROBLEMS

A measurement apparatus, according to a first aspect of the present invention, that measures an internal state parameter of a measurement target, the measurement apparatus including: a contact unit included in a measurement unit that contacts the measurement target; a storage unit that stores reference data indicating a relationship between (i) reference position time series data indicating a position of the contact unit while the contact unit is being pressed into a reference object, (ii) reference pressure time series data indicating pressure applied to the contact unit while the contact unit is being pressed into the reference object, and (iii) an internal state parameter of the reference object; a data acquisition unit that acquires position time series data indicating a position of the contact unit during a measurement period when the contact unit is pressed into the measurement target and pressure time series data indicating pressure applied to the contact unit during the measurement period; and a state identification unit that identifies an internal state parameter of the measurement target by analyzing the position time series data and the pressure time series data using the reference data.

The state identification unit may output, as the internal state parameter, information indicating (i) a distribution of hardness within the measurement target, (ii) a distribution of viscosity within the measurement target, or (iii) a thickness of each of a plurality of layers within the measurement target.

The measurement target may have a plurality of layers, each having different properties, the data acquisition unit may acquire the position time series data and the pressure time series data during a measurement period when the contact unit is being pressed in a direction not parallel to the plurality of layers, and the state identification unit may identify an internal state parameter of the measurement target in association with a position at which the contact unit is pressed into the measurement target.

The storage unit may store, as the reference data, a machine learning model that outputs an internal state parameter of the measurement target when the position time series data and the pressure time series data are input, and
the state identification unit may input the position time series data and the pressure time series data to the machine learning model, and identify an internal state parameter output from the machine learning model as the internal state parameter of the measurement target.

The measurement target may have a plurality of layers, each having different properties, and the machine learning model may be created by deep learning using a plurality of pieces of training data indicating a relationship between the pressure time series data and the position time series data in a plurality of reference objects that each combine a plurality of members corresponding to the plurality of layers and have different physical properties.

The storage unit may store a plurality of sets of the reference data, respectively corresponding to a plurality of the reference objects having different internal state parameters, and the state identification unit may identify, as the internal state parameter of the measurement target, an internal state parameter corresponding to a set of the reference data that is closest to a relationship between the position time series data and the pressure time series data among the plurality of sets of reference data.

The measurement apparatus may further include a state evaluation unit that identifies information regarding an internal state of the measurement target on the basis of the internal state parameter.

The measurement target may be a food, and the information regarding the internal state may be information regarding any one or more of chewiness, putrefaction state, degree of ripeness, and juiciness of the food.

The measurement unit may include a plurality of contact units disposed in a direction orthogonal to a direction in which the contact units are pressed into the measurement target and simultaneously contacting different portions of the measurement target, the data acquisition unit may acquire a plurality of pieces of position time series data indicating positions of the plurality of contact units in a measurement period when the plurality of contact units are pressed into the measurement target, and a plurality of pieces of pressure time series data indicating pressure applied to each of the plurality of contact units in the measurement period, and the state identification unit may identify an internal state parameter of the measurement target by analyzing the plurality of pieces of position time series data and the plurality of pieces of pressure time series data.

The measurement unit may include a plurality of contact units disposed on a surface of a rotating drum and sequentially contacting different portions of the measurement target as the rotating drum rotates, the data acquisition unit may acquire a plurality of pieces of position time series data indicating positions of the plurality of contact units during a measurement period, when the plurality of contact units are sequentially pressed into the measurement target while the rotating drum rotates, and a plurality of pieces of pressure time series data indicating pressure applied to each of the plurality of contact units in the measurement period, and the state identification unit identifies an internal state parameter of the measurement target by analyzing the plurality of pieces of position time series data and the plurality of pieces of pressure time series data.

A measurement method, according to a second aspect of the present invention, implemented by a computer, the method including the steps of: acquiring position time series data indicating a position of a contact unit during a measurement period when the contact unit is pressed into a measurement target, and pressure time series data indicating pressure applied to the contact unit during the measurement period, the contact unit being included in a measurement unit that contacts the measurement target; and identifying an internal state parameter of the measurement target by analyzing the position time series data and the pressure time series data using reference data indicating a relationship between (i) reference position time series data indicating a position of the contact unit while the contact unit is being pressed into a reference object, (ii) reference pressure time series data indicating pressure applied to the contact unit while the contact unit is being pressed into the reference object, and (iii) an internal state parameter of the reference object.

A program, according to a third aspect of the present invention, for making a computer perform: acquiring position time series data indicating a position of a contact unit during a measurement period when the contact unit is pressed into a measurement target, and pressure time series data indicating pressure applied to the contact unit during the measurement period, the contact unit being included in a measurement unit that contacts the measurement target; and identifying an internal state parameter of the measurement target by analyzing the position time series data and the pressure time series data using reference data indicating a relationship between (i) reference position time series data indicating a position of the contact unit while the contact unit is being pressed into a reference object, (ii) reference pressure time series data indicating pressure applied to the contact unit while the contact unit is being pressed into the reference object, and (iii) an internal state parameter of the reference object.

A measurement apparatus, according to a fourth aspect of the present invention, that measures an internal state parameter of a measurement target, the measurement apparatus including: a contact unit included in a measurement unit that contacts the measurement target; a storage unit that stores an arithmetic expression indicating a relationship between (i) position time series data indicating a position of the contact unit while the contact unit is being pressed into an object, (ii) pressure time series data indicating pressure applied to the contact unit while the contact unit is being pressed into the object, and (iii) an internal state parameter of the object; a data acquisition unit that acquires position time series data indicating a position of the contact unit during a measurement period when the contact unit is pressed into the measurement target, and pressure time series data indicating pressure applied to the contact unit during the measurement period; and a state identification unit that identifies an internal state parameter of the measurement target by inputting the position time series data and the pressure time series data into the arithmetic expression.

A measurement method, according to a fifth aspect of the present invention, implemented by a computer, the method including the steps of: acquiring position time series data indicating a position of a contact unit during a measurement period when the contact unit is pressed into a measurement target, and pressure time series data indicating pressure applied to the contact unit during the measurement period, the contact unit being included in a measurement unit that contacts the measurement target; and identifying an internal state parameter of the measurement target by inputting the pressure time series data and the position time series data into an arithmetic expression indicating a relationship between (i) position time series data indicating a position of the contact unit while the contact unit is being pressed into an object, (ii) pressure time series data indicating pressure applied to the contact unit while the contact unit is being pressed into the object, and (iii) an internal state parameter of the object.

A program according to a sixth aspect of the present invention, for making a computer perform, acquiring position time series data indicating a position of a contact unit during a measurement period when the contact unit is pressed into the measurement target, and pressure time series data indicating pressure applied to the contact unit during the measurement period, the contact unit being included in a measurement unit that contacts the measurement target; and identifying an internal state parameter of the measurement target by inputting the pressure time series data and the position time series data into an arithmetic expression indicating a relationship between (i) position time series data indicating a position of the contact unit while the contact unit is being pressed into an object, (ii) pressure time series data indicating pressure applied to the contact unit while the contact unit is being pressed into the object, and (iii) an internal state parameter of the object.

### EFFECT OF THE INVENTION

According to the present invention, it is possible to identify the distribution of the internal state of a measurement target.

### BRIEF DESCRIPTION OF THE DRAWINGS

FIG. 1 is a diagram illustrating an overview of a measurement apparatus S.
FIG. 2 is a diagram showing the change in pressure when, after moving toward a measurement target W, a contact unit 11 moves away from the measurement target W.
FIG. 3 is a diagram illustrating the configuration of an analysis unit 2.
FIG. 4 is an example of data indicating the relationship between position and pressure.
FIG. 5 is a flowchart illustrating the processing flow in the analysis unit 2.
FIG. 6 is a diagram illustrating the configuration of a measurement unit 1A according to a first modification.
FIG. 7 is a diagram illustrating a configuration of a measurement unit 1B according to a second modification.

### DETAILED DESCRIPTION OF THE INVENTION

### [Overview of a measurement apparatus S]

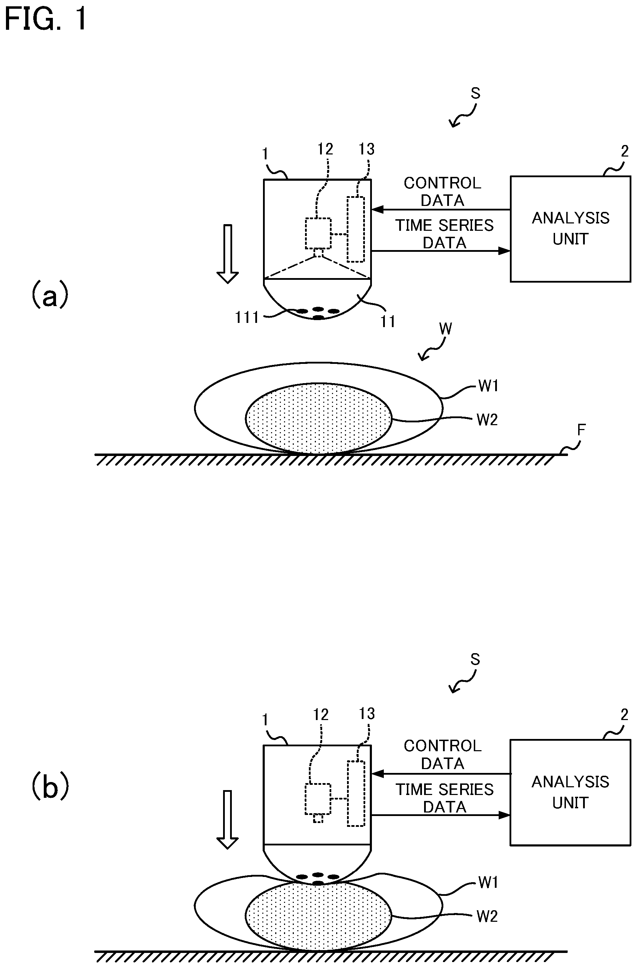

FIG. 1 is a diagram illustrating an example overview of a measurement apparatus S. The measurement apparatus S is an apparatus designed to measure internal state parameters of a measurement target W that is placed on a floor F. The internal state parameters represent data indicating the distribution of physical properties of the materials constituting the measurement target W. Examples of such parameters include the distribution of hardness or viscosity within the measurement target W. When the interior of the measurement target W is composed of a plurality of layers, the internal state parameters include data indicating the thicknesses of the respective layers.

The measurement target W may be any object; however, an object having a nonhomogeneous interior, in which hardness or viscosity varies depending on position, is preferable as the measurement target W. Examples of the measurement target W include food products such as bread, fruits, and vegetables, (e.g., deep-fried tofu, tofu, and thin fried tofu), clothing, and food products or industrial goods enclosed in packaging materials.

The measurement apparatus S includes a measurement unit 1 and an analysis unit 2. The measurement unit 1 moves on the basis of control data transmitted from the analysis unit 2, and, while pressing against the measurement target W, measures the pressure received by the measurement unit 1 to generate (i) position time series data indicating the position of the measurement unit 1, and (ii) pressure time series data indicating the pressure received by the measurement unit 1 In the position time series data, time is associated with the position of the measurement unit 1. In the pressure time series data, time is associated with the pressure received by the measurement unit 1. The measurement unit 1 transmits the generated position time series data and the generated pressure time series data indicating the pressure, to the analysis unit 2.

The analysis unit 2 includes, for example, a processor, and identifies an internal state parameter of the measurement target W by analyzing the position time series data and the pressure time series data received from the measurement unit 1. The analysis unit 2 outputs, as the identified internal state parameter, information indicating the distribution of hardness or viscosity within the measurement target W. The analysis unit 2 may identify a layer configuration inside the measurement target W on the basis of the distribution of hardness or viscosity within the measurement target W, and output information indicating the positions of a plurality of identified layers and the thicknesses of the respective layers.

The measurement unit 1 includes a contact unit 11, an imaging unit 12, and a control unit 13. The contact unit 11 is a part that comes into contact with the measurement target W, and includes one or more contact members 111. The contact member 111 may have any configuration as long as it can detect pressure it receives. However, in the present embodiment, the contact member 111 is an object that deforms upon receiving pressure.

The imaging unit 12 generates captured image data by capturing the contact member 111, and inputs the generated captured image data to the control unit 13. The control unit 13 includes, for example, a processor, and identifies the pressure received by the contact member 111 on the basis of the shape or size of an image of the contact member 111 included in the captured image data. The control unit 13 identifies the pressure on the basis of the shape or size of the image of the contact member 111 by referencing, for example, data stored in a memory in which the shape or size of the contact member 111 and the pressure are associated. The control unit 13 generates pressure time series data in which the time output from a timekeeping unit and the pressure are associated. The control unit 13 transmits the pressure time series data to the analysis unit 2.

As illustrated in FIG. 1, when the contact unit 11 includes the plurality of contact members 111, the control unit 13 identifies the pressure applied to each of the plurality of contact members 111. In this case, the control unit 13 may transmit the pressure time series data associated with each of the plurality of contact members 111 to the analysis unit 2.

The control unit 13 further identifies the position of the measurement unit 1. For example, the measurement unit 1 is coupled to a fixed member (for example, a linear actuator) in a state in which the measurement unit 1 is movable, and the control unit 13 identifies a position in a movement direction of the measurement unit 1 on the basis of data output from the linear encoder included in the fixed member. The movement direction of the measurement unit 1 is, for example, the direction toward the center position of the measurement target W (for example, the vertical direction in FIG. 1), but may be another direction. The control unit 13 generates position time series data in which the time, output from the timekeeping unit, and the position are associated. The control unit 13 transmits the position time series data to the analysis unit 2. When the contact unit 11 includes the plurality of contact members 111, the control unit 13 may transmit position time series data associated with each of the plurality of contact members 111 to the analysis unit 2.

FIG. 1(a) shows a state in which the contact unit 11 is not in contact with the measurement target W, and FIG. 1(b) shows a state in which the measurement unit 1 has moved toward the measurement target W from the state of FIG. 1(a) and is pressing the measurement target W. For example, the control unit 13 transmits, to the analysis unit 2, the pressure time series data and the position time series data generated after and including a point in time at which the contact unit 11 comes into contact with the measurement target W.

In the example illustrated in FIG. 1, the measurement target W includes a packaging material W1 and a contained object W2 accommodated in the packaging material W1. A gas (for example, air) is sealed between the packaging material W1 and the contained object W2. When the measurement unit 1 presses such a measurement target W, the pressure received by the contact unit 11 differs between a state in which the contact unit 11 is not in contact with the contained object W2 through the packaging material W1 and a state in which the contact unit 11 is in contact with the contained object W2 through the packaging material W1. Furthermore, in the case where the interior of the contained object W2 is not homogeneous, the pressure received by the contact unit 11 also differs depending on the amount by which the contact unit 11 presses into the contained object W2.

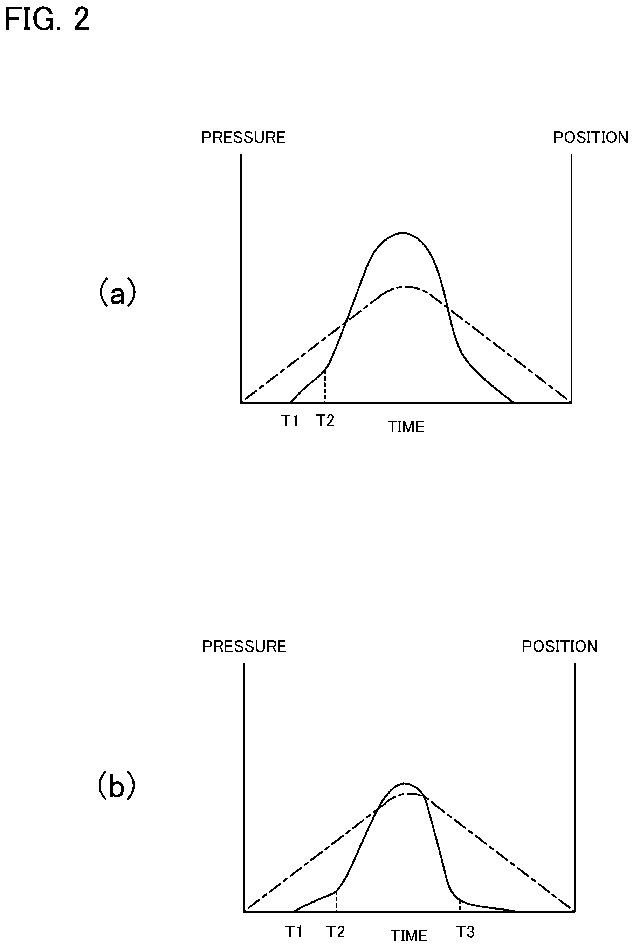

FIG. 2 is a diagram showing the change in pressure when, after moving toward the measurement target W, the contact unit 11 moves away from the measurement target W. The solid line indicates the pressure received by the contact unit 11, and the one-dot chain line indicates the position in the movement direction of the measurement unit 1. FIG. 2(a) shows a case in which the packaging material W1 is sealed, whereas FIG. 2(b) shows a case in which the packaging material W1 has a hole and air escapes when the packaging material W1 is pressed.

FIG. 2 shows the pressure received by the contact unit 11 from a point in time at which the contact unit 11 is not in contact with the measurement target W, as shown in FIG. 1(a). Therefore, the pressure is zero from the time the measurement unit 1 begins moving until time T1. As shown in FIG. 2(a), after the contact unit 11 comes into contact with the packaging material W1 at time T1, the pressure begins to rise. From time T2, when the contact unit 11 comes into contact with the contained object W2 through the packaging material W1, the rate of pressure increase becomes faster.

In the example shown in FIG. 2(b), the pressure is smaller than that in the example shown in FIG. 2(a) because the air escapes as the contact unit 11 presses the packaging material W1. After time T3, at which the contact unit 11 is no longer in contact with the contained object W2 through the packaging material W1 due to the escape of air, the pressure received by the contact unit 11 is smaller than the pressure in the example shown in FIG. 2(a).

As described above, the manner in which pressure changes over time differs depending on the internal state of the measurement target W. Accordingly, the analysis unit 2 is capable of identifying the internal state of the measurement target W by analyzing the pressure time series data.

### [Configuration of the analysis unit 2]

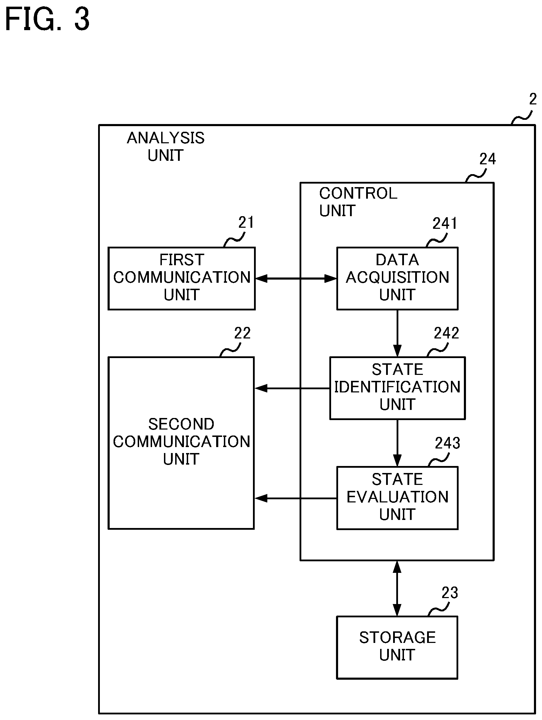

FIG. 3 is a diagram illustrating an example of a configuration of the analysis unit 2. The analysis unit 2 includes a first communication unit 21, a second communication unit 22, a storage unit 23, and a control unit 24. The control unit 24 includes a data acquisition unit 241, a state identification unit 242, and a state evaluation unit 243.

The first communication unit 21 has a communication interface for transmitting and receiving data to and from the measurement unit 1. The first communication unit 21 may transmit and receive data to and from the measurement unit 1 by wireless communication or may transmit and receive data to and from the measurement unit 1 by wired communication. The first communication unit 21 transmits control data input from the data acquisition unit 241 to the measurement unit 1. The first communication unit 21 inputs the position time series data and the pressure time series data received from the measurement unit 1 to the data acquisition unit 241.

The second communication unit 22 includes a communication interface for transmitting data input from the state identification unit 242 to an external device, such as a computer or a printer. For example, the second communication unit 22 transmits to the external device the internal state parameter of the measurement target W identified by the state identification unit 242, and information indicating the distribution of physical properties within the measurement target W identified on the basis of the internal state parameter.

The storage unit 23 includes a storage medium such as a read only memory (ROM), a random access memory (RAM), and a solid state drive (SSD). The storage unit 23 stores programs executed by the control unit 24. In addition, the storage unit 23 stores the position time series data and the pressure time series data transmitted from the measurement unit 1 via the first communication unit 21.

Furthermore, the storage unit 23 stores reference data indicating the relationship between (i) reference position time series data indicating the position of the contact unit 11 while the contact unit 11 is being pressed into a reference object, (ii) reference pressure time series data indicating the pressure applied to the contact unit 11 while the contact unit 11 is being pressed into the reference object, and (iii) internal state parameters of the reference object. That is, the storage unit 23 stores reference data indicating the relationship between the position of the contact unit 11 and the pressure applied to the contact unit 11 while the contact unit 11 is being pressed into a reference object whose internal state parameters are known. The position of the contact unit 11 is, for example, a position in the direction in which the contact unit 11 is pressed into the measurement target W. The storage unit 23 stores, for example, a plurality of sets of reference data corresponding to a plurality of reference objects having different internal state parameters.

The reference object is, for example, an object formed by combining a plurality of materials having known physical properties, and the reference data is time series data indicating the relationship between position, pressure, and time measured while the contact unit 11 is being pressed into the object. The reference position time series data is time series data of the position measured while the contact unit 11 is being pressed into the reference object, and the reference pressure time series data is time series data of the pressure measured while the contact unit 11 is being pressed into the reference object. The reference object is, for example, an object having a multilayer structure formed by stacking a plurality of members having different hardness, viscosity, and thickness. The reference object is an object having known regions in which each of the plurality of members exists.

The reference object need not be an actual object, and may be a virtual three-dimensional model. In this case, the reference data may be time series data indicating the relationship between position, pressure, and time calculated by simulating the movement in which the contact unit 11 is pressed into the three-dimensional model of the object.

The storage unit 23 may store an arithmetic expression indicating the relationship between the position time series data and pressure time series data measured while the contact unit 11 is being pressed into the object, and the internal state parameters of the object. The object to which the arithmetic expression applies is preferably of the same type as the measurement target. A known method or expression may be used as the arithmetic expression. For example, when the hardness (degree of hardness) is obtained as the internal state parameter, the elastic modulus can be obtained from the position time series data and the pressure time series data, and the elastic modulus can be converted into the hardness (degree of hardness) by a known conversion method.

The storage unit 23 may store, as reference data, a machine learning model that outputs internal state parameters of the measurement target W when the position time series data and the pressure time series data are input. That is, the reference data may be a machine learning model created by learning using (i) the position time series data and the pressure time series data indicating the relationship between position, pressure, and time measured while the contact unit 11 is being pressed into a large number of objects having different internal state parameters, and (ii) the internal state parameters as training data. The machine learning model may also be created by learning using (i) the position time series data and the pressure time series data calculated by simulating the movement in which the contact unit 11 is pressed into the three-dimensional model and (ii) the internal state parameters as training data.

When the measurement target W has a plurality of layers, each having different properties, the machine learning model may be created by deep learning using a plurality of pieces of training data indicating the relationship between the position time series data and the pressure time series data in a plurality of reference objects that each combine a plurality of members corresponding to the plurality of layers and have different physical properties. Since a large number of reference objects having different internal state parameters can be easily created by changing the combinations of members corresponding to the plurality of layers, learning can be efficiently performed using a large number of pieces of training data by employing such reference objects.

When the contact unit 11 includes the plurality of contact members 111 as illustrated in FIG. 1, the storage unit 23 may store a plurality of sets of reference data in association with the plurality of contact members 111.

The control unit 24 includes, for example, a CPU (Central Processing Unit). The control unit 24 functions as the data acquisition unit 241, the state identification unit 242, and the state evaluation unit 243 by executing the programs stored in the storage unit 23.

The data acquisition unit 241 acquires, via the first communication unit 21, the position time series data indicating the position of the contact unit 11 during a measurement period when the contact unit 11 included in the measurement unit 1 is pressed into the measurement target W, and the pressure time series data indicating the pressure applied to the contact unit 11 during the measurement period. The measurement period is, for example, a period from when the contact unit 11 comes into contact with the measurement target W to when the contact unit 11 separates from the measurement target W. When the contact unit 11 includes the plurality of contact members 111 as illustrated in FIG. 1, the data acquisition unit 241 acquires a plurality of pieces of position time series data and a plurality of pieces of pressure time series data associated with each of the plurality of contact members 111.

The position time series data and the pressure time series data may include position data and pressure data for a period in which the contact unit 11 is not in contact with the measurement target W. The position time series data and the pressure time series data may include, for example, position data and pressure data for a period from the initial position of the measurement unit 1, as the measurement unit 1 moves toward the measurement target W and reaches a predetermined position, until it returns to the initial position.

When the measurement target W has a plurality of layers, each having different properties, the data acquisition unit 241 acquires the position time series data and the pressure time series data while the contact unit 11 is being pressed in a direction not parallel to the plurality of layers.

The state identification unit 242 identifies an internal state parameter of the measurement target W corresponding to the relationship between the position time series data and the pressure time series data, using the reference data stored in the storage unit 23. The state identification unit 242 identifies the internal state parameter of the measurement target W in association with the position at which the measurement target W is pressed.

For example, the state identification unit 242 associates the position and pressure at the same time in the position time series data and the pressure time series data acquired by the data acquisition unit 241 to create data indicating the relationship between the position of the contact unit 11 and the pressure received by the contact unit 11, and identifies the internal state parameter of the measurement target W on the basis of the created data. When the contact unit 11 includes a plurality of contact members 111, the state identification unit 242 may identify the internal state parameter of the measurement target W on the basis of a plurality of pieces of position time series data and a plurality of pieces of pressure time series data corresponding to the contact members 111, using reference data corresponding to the plurality of contact members 111.

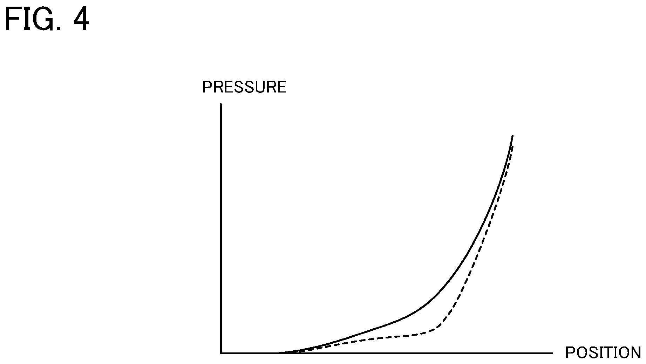

FIG. 4 is an example of data indicating the relationship between position and pressure. FIG. 4 corresponds to the position time series data and the pressure time series data shown in FIG. 2. The solid line corresponds to the position time series data and the pressure time series data shown in FIG. 2(a), and the broken line corresponds to the position time series data and the pressure time series data shown in FIG. 2(b). As can be seen from a comparison between the solid line and the broken line, the relationship between the position and the pressure varies depending on the internal state of the measurement target W. For example, the state identification unit 242 may identify the internal state parameter by analyzing the graph of the measured position and pressure, or may identify the internal state parameter of the measurement target W corresponding to the relationship between the position time series data and the pressure time series data by using the reference data stored in the storage unit 23.

The state identification unit 242 identifies, as the internal state parameter of the measurement target W, an internal state parameter corresponding to the reference data that is closest to the relationship between the position time series data and the pressure time series data among the plurality of sets of reference data. For example, the state identification unit 242 calculates a correlation value between the reference data and the data indicating the relationship between the position and the pressure based on the measurement result, and determines that the internal state parameter associated with the reference data having the largest correlation value is the internal state parameter of the measurement target W.

The state identification unit 242 may input the position time series data and the pressure time series data to the machine learning model, and identify the internal state parameter output from the machine learning model as the internal state parameter of the measurement target W.

The state identification unit 242 may output information, identified as the internal state parameter of the measurement target W, indicating the distribution of hardness or viscosity within the measurement target W, or the thicknesses of the respective layers within the measurement target W. For example, the state identification unit 242 outputs image data or text data indicating the distribution of the hardness level and the viscosity level within the measurement target W by referencing data indicating the relationship between the internal state parameter and the hardness level or the viscosity level. The state identification unit 242 creates data indicating the distribution of the hardness level and the viscosity level within the measurement target W by identifying the hardness level or viscosity level to which the internal state parameter of hardness or viscosity, corresponding to each of the plurality of positions of the measurement target W, belongs.

The state identification unit 242 may output a result of identifying the thicknesses of the respective layers, on the assumption that a position at which the amount of change in hardness or viscosity indicated by the internal state parameter is equal to or greater than a threshold value is a boundary position between the plurality of layers. The state identification unit 242 may also output data indicating the substance of each of the layers by referencing data indicating the relationship between the internal state parameter and the type of the substance. Since the state identification unit 242 outputs such data, the user of the measurement apparatus S can easily grasp the internal structure of the measurement target W.

The state identification unit 242 may identify an internal state parameter of the measurement target by inputting the position time series data and the pressure time series data acquired by the data acquisition unit 241 into the arithmetic expression stored in the storage unit 23.

The state evaluation unit 243 identifies information regarding the internal state of the measurement target W on the basis of the internal state parameters. The state evaluation unit 243 outputs the identified information via the second communication unit 22. For example, when the measurement target W is food, the state evaluation unit 243 can identify information regarding the internal state of the food on the basis of internal state parameters (hardness, viscosity, and the like).

When the food is a fruit, the state evaluation unit 243 identifies, as information regarding the internal state, one or more of, for example, chewiness, putrefaction state, degree of ripeness, and juiciness, represented by evaluation indexes of 1 to 5. In addition, when the food is deep-fried tofu, the state evaluation unit 243 may identify information regarding the internal state, such as chewiness or putrefaction state, represented by evaluation indexes of 1 to 5. Furthermore, when the food is thin fried tofu (for inari sushi), the state evaluation unit 243 may identify, as information regarding the internal state, properties such as resistance to tearing, represented by evaluation indexes of 1 to 5. The state evaluation unit 243 may, in outputting information regarding the internal state (chewiness, putrefaction state, degree of ripeness, juiciness, resistance to tearing) as evaluation indexes of 1 to 5, use either a rule-based algorithm or a trained machine learning model.

### [Processing flow in the analysis unit 2]

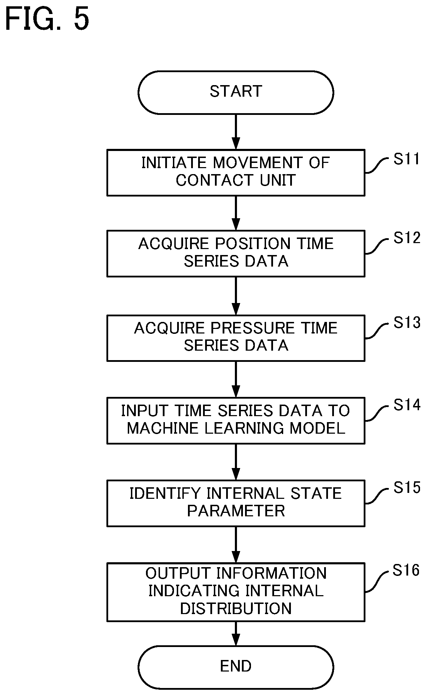

FIG. 5 is a flowchart illustrating an example of the processing flow in the analysis unit 2. The flowchart shown in FIG. 5 starts from the point at which the measurement of the internal state parameters of one measurement target W is initiated.

The data acquisition unit 241 transmits control data for moving the contact unit 11 to the measurement unit 1, and initiates the movement of the contact unit 11 toward the measurement target W (S11). Thereafter, the data acquisition unit 241 acquires the position time series data and the pressure time series data generated by the measurement unit 1 (S12, S13). The data acquisition unit 241 may acquire the position time series data and the pressure time series data corresponding to the measurement period after the measurement by the measurement unit 1 has ended, or may acquire the position data and the pressure data multiple times at predetermined time intervals.

Upon the data acquisition unit 241 acquiring the position time series data and the pressure time series data, the state identification unit 242 analyzes the position time series data and the pressure time series data. For example, the data acquisition unit 241 inputs the position time series data and the pressure time series data to a machine learning model (S14), and identifies an internal state parameter of the measurement target W from the data output by the machine learning model (S15). Next, the state identification unit 242 outputs information indicating the distribution of hardness and viscosity within the measurement target W, or the layer configuration within the measurement target W, as the identified internal state parameter (S16).

Although not shown in FIG. 5, the state evaluation unit 243 may output information on the internal state of the measurement target W on the basis of the internal state parameter following the output of the information on the internal state parameter (S16). The above-described method can be used as a specific example of the information related to the internal state of the measurement target W and a method of generating or outputting this information.

### [First Modification]

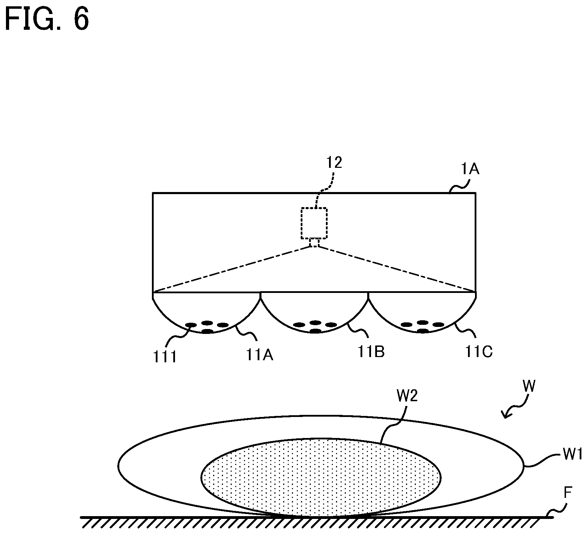

FIG. 6 is a diagram illustrating the configuration of a measurement unit 1A according to a first modification. The measurement unit 1A is different from the measurement unit 1 shown in FIG. 1 in that it has a plurality of contact units 11 (contact unit 11A, contact unit 11B, contact unit 11C), and is otherwise the same. The plurality of contact units 11 are disposed, for example, in a direction orthogonal to the pressing direction of the measurement unit 1, and simultaneously contact different portions of the measurement target W. The plurality of contact units 11 may be disposed on a curve having the same shape as the outline of the measurement target W.

Here, when the storage unit 23 is used, the storage unit 23 stores reference data indicating the relationship between the position of each of the plurality of contact units 11 and the pressure applied to each of the plurality of contact units 11 while the plurality of contact units 11 are being pressed into a reference object whose internal state parameters are known. The data acquisition unit 241 acquires a plurality of pieces of position time series data indicating the position of each of the plurality of contact units 11 in the pressing direction of the measurement unit 1 during a measurement period when the plurality of contact units 11 are pressed into the measurement target W, and a plurality of pieces of pressure time series data indicating the pressure applied to each of the plurality of contact units 11 in the measurement period.

The state identification unit 242 analyzes the plurality of pieces of position time series data and the plurality of pieces of pressure time series data acquired by the data acquisition unit 241 to identify an internal state parameter of the measurement target W. When the storage unit 23 is used, the state identification unit 242 identifies the internal state parameter of the measurement target W corresponding to the relationship between the plurality of pieces of position time series data and the plurality of pieces of pressure time series data by using the reference data stored in the storage unit 23. The state identification unit 242 may identify the internal state parameter using a plurality of pieces of data indicating the relationship between the positions and the pressures included in the reference data, or may identify the internal state parameter by inputting the plurality of pieces of position time series data and the plurality of pieces of pressure time series data to the reference data when the reference data is a machine learning model.

As shown in FIG. 6, when the measurement unit 1 includes the plurality of contact units 11, each contact unit 11 receives pressure in directions other than its pressing direction because the measurement target W, deformed by other contact units 11 pressing the measurement target W, presses the contact unit 11. Since each of the plurality of contact units 11 has a plurality of contact members 111 and the imaging unit 12 captures the shapes of the plurality of contact members 111 in the plurality of contact units 11, a plurality of pressures applied to the contact units 11 from multiple directions at multiple positions of the measurement target W can be measured.

In this case, the control unit 13 transmits to the analysis unit 2 the time series data indicating the relationship between the position and the pressure corresponding to each of the plurality of contact members 111, and the data acquisition unit 241 acquires the plurality of pieces of position time series data and the plurality of pieces of pressure time series data associated with the plurality of contact members 111 of each of the plurality of contact units 11. The state identification unit 242 identifies the internal state parameter of the measurement target W on the basis of the reference data corresponding to the plurality of contact members 111, the plurality of pieces of position time series data, and the plurality of pieces of pressure time series data. Because the measurement unit 1 and the analysis unit 2 have such a configuration, the state identification unit 242 can identify the internal state parameter on the basis of time series data of pressures at many positions of the measurement target W, allowing the internal state parameter to be identified across a wide range of the measurement target W.

### [Second Modification]

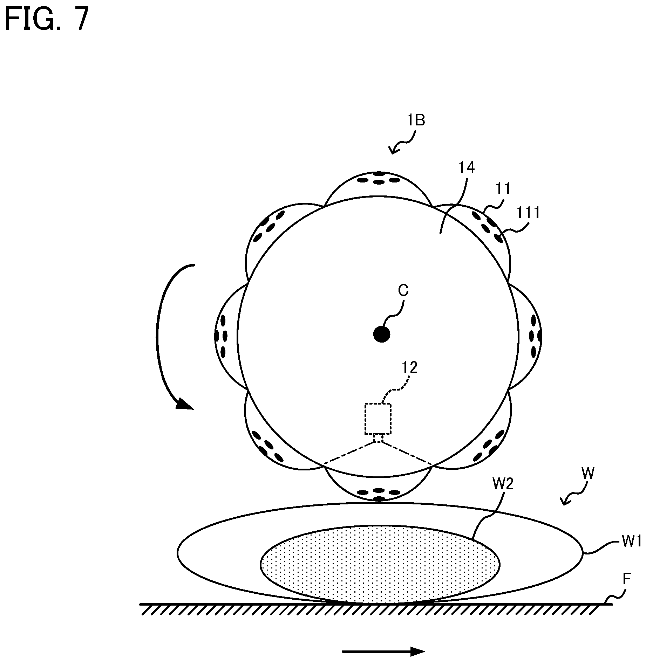

FIG. 7 is a diagram illustrating a configuration of a measurement unit 1B according to a second modification. The measurement unit 1B includes a rotating drum 14 and a plurality of contact units 11 that sequentially contact different portions of the measurement target W as the rotating drum 14 rotates about the rotation axis C. The imaging unit 12 captures the contact member 111 in the contact unit 11, which does not rotate with the rotation of the rotating drum 14, and comes into contact with the measurement target W. As one example, during the measurement period, the rotating drum 14 rotates together with movement of the floor F.

The data acquisition unit 241 acquires a plurality of pieces of position time series data indicating the positions of the plurality of contact units 11 during the measurement period when the plurality of contact units 11 are sequentially pressed into the measurement target W while the rotating drum 14 rotates, and a plurality of pieces of pressure time series data indicating the pressure applied to each of the plurality of contact units 11 in the measurement period. As in the first modification, the state identification unit 242 analyzes the plurality of pieces of position time series data and the plurality of pieces of pressure time series data to identify an internal state parameter of the measurement target W. As in the first modification, the state identification unit 242 can also identify the internal state parameter of the measurement target W corresponding to the relationship between the plurality of pieces of position time series data and the plurality of pieces of pressure time series data by using the reference data.

The data acquisition unit 241 may acquire a plurality of pieces of position time series data and a plurality of pieces of pressure time series data associated with a plurality of contact members 111 included in each of the plurality of contact units 11. In this case, the state identification unit 242 may identify an internal state parameter of the measurement target W on the basis of the reference data corresponding to the plurality of contact members 111, the plurality of position time series data, and the plurality of pressure time series data.

Since the measurement unit 1 and the analysis unit 2 are configured in this way, the state identification unit 242 can identify the internal state parameter using the position time series data and the pressure time series data obtained when the plurality of contact units 11 respectively contact different positions of the measurement target W at different times. Therefore, even when the lateral width of the measurement unit 1 is smaller than the measurement unit 1, the internal state parameter can be efficiently identified across a wide range of the measurement target W.

### [Effects by the measurement apparatus S]

As described above, the measurement unit 1 includes the data acquisition unit 241 that acquires the position time series data indicating the position of the contact unit 11 during the measurement period when the contact unit 11 of the measurement unit 1 that contacts the measurement target W is pressed into the measurement target W and the pressure time series data indicating the pressure applied to the contact unit 11 during the measurement period. The state identification unit 242 identifies the internal state parameter of the measurement target W corresponding to the relationship between the pressure time series data and the position time series data, using the reference data stored in the storage unit 23. Accordingly, the measurement apparatus S can identify the internal state of the measurement target W, such as the distribution of physical properties within the measurement target W or whether a hole or a tear is present in the measurement target W, with high accuracy.

The present invention is explained based on the exemplary embodiments. The technical scope of the present invention is not limited to the scope explained in the above embodiments and it is possible to make various changes and modifications within the scope of the disclosure. For example, in the above description, a case where the measurement target W includes the packaging material W1 and the contained object W2 is exemplified, but the measurement target W does not need to include the packaging material W1.

All or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

### [Description of Symbols]

1 Measurement unit
2 Analysis unit
11 Contact unit
12 Imaging unit
13 Control unit
14 Rotating drum
21 First communication unit
22 Second communication unit
23 Storage unit
24 Control unit
111 Contact member
241 Data acquisition unit
242 State identification unit
243 State evaluation unit

## Claims

1. A measurement apparatus that measures an internal state parameter of a measurement target, the measurement apparatus comprising:
a contact unit included in a measurement unit that contacts the measurement target;
a storage unit that stores reference data indicating a relationship between (i) reference position time series data indicating a position of the contact unit while the contact unit is being pressed into a reference object, (ii) reference pressure time series data indicating pressure applied to the contact unit while the contact unit is being pressed into the reference object, and (iii) an internal state parameter of the reference object;
a data acquisition unit that acquires position time series data indicating a position of the contact unit during a measurement period when the contact unit is pressed into the measurement target and pressure time series data indicating pressure applied to the contact unit during the measurement period; and
a state identification unit that identifies an internal state parameter of the measurement target by analyzing the position time series data and the pressure time series data using the reference data.

2. The measurement apparatus according to claim 1, wherein the state identification unit outputs, as the internal state parameter, information indicating (i) a distribution of hardness within the measurement target, (ii) a distribution of viscosity within the measurement target, or (iii) a thickness of each of a plurality of layers within the measurement target.

3. The measurement apparatus according to claim 1, wherein the measurement target has a plurality of layers, each having different properties,
the data acquisition unit acquires the position time series data and the pressure time series data during a measurement period when the contact unit is being pressed in a direction not parallel to the plurality of layers, and
the state identification unit identifies an internal state parameter of the measurement target in association with a position at which the contact unit is pressed into the measurement target.

4. The measurement apparatus according to claim 1, wherein the storage unit stores, as the reference data, a machine learning model that outputs an internal state parameter of the measurement target when the position time series data and the pressure time series data are input, and
the state identification unit inputs the position time series data and the pressure time series data to the machine learning model, and identifies an internal state parameter output from the machine learning model as the internal state parameter of the measurement target.

5. The measurement apparatus according to claim 4, wherein the measurement target has a plurality of layers, each having different properties, and
the machine learning model is created by deep learning using a plurality of pieces of training data indicating a relationship between the pressure time series data and the position time series data in a plurality of reference objects that each combine a plurality of members corresponding to the plurality of layers and have different physical properties.

6. The measurement apparatus according to claim 1, wherein the storage unit stores a plurality of sets of the reference data, respectively corresponding to a plurality of the reference objects having different internal state parameters, and
the state identification unit identifies, as the internal state parameter of the measurement target, an internal state parameter corresponding to a set of the reference data that is closest to a relationship between the position time series data and the pressure time series data among the plurality of sets of reference data.

7. The measurement apparatus according to claim 1, wherein the measurement apparatus further includes a state evaluation unit that identifies information regarding an internal state of the measurement target on the basis of the internal state parameter.

8. The measurement apparatus according to claim 7, wherein the measurement target is a food, and the information regarding the internal state is information regarding any one or more of chewiness, putrefaction state, degree of ripeness, and juiciness of the food.

9. The measurement apparatus according to claim 1, wherein the measurement unit includes a plurality of contact units disposed in a direction orthogonal to a direction in which the contact units are pressed into the measurement target and simultaneously contacting different portions of the measurement target,
the data acquisition unit acquires a plurality of pieces of position time series data indicating positions of the plurality of contact units in a measurement period when the plurality of contact units are pressed into the measurement target, and a plurality of pieces of pressure time series data indicating pressure applied to each of the plurality of contact units in the measurement period, and
the state identification unit identifies an internal state parameter of the measurement target by analyzing the plurality of pieces of position time series data and the plurality of pieces of pressure time series data.

10. The measurement apparatus according to claim 1, wherein the measurement unit includes a plurality of contact units disposed on a surface of a rotating drum and sequentially contacting different portions of the measurement target as the rotating drum rotates,
the data acquisition unit acquires a plurality of pieces of position time series data indicating positions of the plurality of contact units during a measurement period, when the plurality of contact units are sequentially pressed into the measurement target while the rotating drum rotates, and a plurality of pieces of pressure time series data indicating pressure applied to each of the plurality of contact units in the measurement period, and
the state identification unit identifies an internal state parameter of the measurement target by analyzing the plurality of pieces of position time series data and the plurality of pieces of pressure time series data.

11. A measurement method implemented by a computer, the method comprising the steps of:
acquiring position time series data indicating a position of a contact unit during a measurement period when the contact unit is pressed into a measurement target, and pressure time series data indicating pressure applied to the contact unit during the measurement period, the contact unit being included in a measurement unit that contacts the measurement target; and
identifying an internal state parameter of the measurement target by analyzing the position time series data and the pressure time series data using reference data indicating a relationship between (i) reference position time series data indicating a position of the contact unit while the contact unit is being pressed into a reference object, (ii) reference pressure time series data indicating pressure applied to the contact unit while the contact unit is being pressed into the reference object, and (iii) an internal state parameter of the reference object.

12. A program for making a computer perform:
acquiring position time series data indicating a position of a contact unit during a measurement period when the contact unit is pressed into a measurement target, and pressure time series data indicating pressure applied to the contact unit during the measurement period, the contact unit being included in a measurement unit that contacts the measurement target; and
identifying an internal state parameter of the measurement target by analyzing the position time series data and the pressure time series data using reference data indicating a relationship between (i) reference position time series data indicating a position of the contact unit while the contact unit is being pressed into a reference object, (ii) reference pressure time series data indicating pressure applied to the contact unit while the contact unit is being pressed into the reference object, and (iii) an internal state parameter of the reference object.

13. A measurement apparatus that measures an internal state parameter of a measurement target, the measurement apparatus comprising:
a contact unit included in a measurement unit that contacts the measurement target;
a storage unit that stores an arithmetic expression indicating a relationship between (i) position time series data indicating a position of the contact unit while the contact unit is being pressed into an object, (ii) pressure time series data indicating pressure applied to the contact unit while the contact unit is being pressed into the object, and (iii) an internal state parameter of the object;
a data acquisition unit that acquires position time series data indicating a position of the contact unit during a measurement period when the contact unit is pressed into the measurement target, and pressure time series data indicating pressure applied to the contact unit during the measurement period; and
a state identification unit that identifies an internal state parameter of the measurement target by inputting the position time series data and the pressure time series data into the arithmetic expression.

14. A measurement method implemented by a computer, the method comprising the steps of:
acquiring position time series data indicating a position of a contact unit during a measurement period when the contact unit is pressed into a measurement target, and pressure time series data indicating pressure applied to the contact unit during the measurement period, the contact unit being included in a measurement unit that contacts the measurement target; and
identifying an internal state parameter of the measurement target by inputting the pressure time series data and the position time series data into an arithmetic expression indicating a relationship between (i) position time series data indicating a position of the contact unit while the contact unit is being pressed into an object, (ii) pressure time series data indicating pressure applied to the contact unit while the contact unit is being pressed into the object, and (iii) an internal state parameter of the object.

15. A program for making a computer perform,
acquiring position time series data indicating a position of a contact unit during a measurement period when the contact unit is pressed into the measurement target, and pressure time series data indicating pressure applied to the contact unit during the measurement period, the contact unit being included in a measurement unit that contacts the measurement target; and
identifying an internal state parameter of the measurement target by inputting the pressure time series data and the position time series data into an arithmetic expression indicating a relationship between (i) position time series data indicating a position of the contact unit while the contact unit is being pressed into an object, (ii) pressure time series data indicating pressure applied to the contact unit while the contact unit is being pressed into the object, and (iii) an internal state parameter of the object.
